# EUROPEAN PATENT SPECIFICATION

(11) **EP 1 474 164 B1**
(45) Date of publication and mention of the grant of the patent: **29.10.2008**
(21) Application number: 03729360.2
(22) Date of filing: 08.01.2003
(51) Int. Cl.: A61K 38/22, A61P 3/06

(54) **USE OF AMYLIN, AMYLIN ANALOGS AND AMYLIN DERIVATIVES TO TREAT DYSLIPIDEMIA AND TRIGLYCERIDEMIA**
VERWENDUNG VON AMYLIN, AMYLINANALOGA UND AMYLIN DERIVATEN ZUR BEHANDLUNG VON DYSLIPIDÄMIE UND HYPERTRIGLYCERIDÄMIE
UTILISATION DE L'AMYLINE, D'ANALOGUES ET DE DÉRIVÉS POUR LE TRAITEMENT DE LA DYSLIPIDÉMIE ET DE L'HYPERTRIGLYCÉRIDÉMIE

(30) Priority: 08.01.2002 US 347128 P
(43) Date of publication of application: 10.11.2004
(73) Proprietor: AMYLIN PHARMACEUTICALS, INC., San Diego, CA 92121 (US)
(72) Inventor: KOLTERMAN, Orville, G., Poway, CA 92064 (US); WEYER, Christian, San Diego, CA 92122 (US); MAGGS, David, G., Del Mar, CA 92014 (US); FINEMAN, Mark, San Diego, CA 92131 (US)
(74) Representative: UEXKÜLL & STOLBERG
(86) International application number: PCT/US2003/000369
(87) International publication number: WO 2003/057244

(56) References cited:
- WO-A-98/30231
- WO-A-98/45313
- WO-A-98/55144
- US-A- 6 087 334
- THOMPSON R G ET AL: "Pramlintide, a synthetic analog of human amylin, improves the metabolic profile of patients with type 2 diabetes using insulin. The Pramlintide in Type 2 Diabetes Group." DIABETES CARE. UNITED STATES JUN 1998, vol. 21, no. 6, June 1998 (1998-06), pages 987-993, XP001147768 ISSN: 0149-5992
- NYHOLM BIRGIT ET AL: "The amylin analog pramlintide improves glycemic control and reduces postprandial glucagon concentrations in patients with type 1 diabetes mellitus." METABOLISM CLINICAL AND EXPERIMENTAL, vol. 48, no. 7, July 1999 (1999-07), pages 935-941, XP009009876 ISSN: 0026-0495
- HETTIARACHCHI M ET AL: "Rat amylin-(8-37) enhances insulin action and alters lipid metabolism in normal and insulin-resistant rats." THE AMERICAN JOURNAL OF PHYSIOLOGY. UNITED STATES NOV 1997, vol. 273, no. 5 Pt 1, November 1997 (1997-11), pages E859-E867, XP002241729 ISSN: 0002-9513
- YE JI-MING ET AL: "Evidence that amylin stimulates lipolysis in vivo: A possible mediator of induced insulin resistance." AMERICAN JOURNAL OF PHYSIOLOGY, vol. 280, no. 4 Part 1, April 2001 (2001-04), pages E562-E569, XP009009879 ISSN: 0002-9513
- DATABASE WPI Section Ch, Week 200261 Derwent Publications Ltd., London, GB; Class A96, AN 2002-567189 XP002241730 & CN 1 342 456 A (CHEN L), 3 April 2002 (2002-04-03)
- WANT L ET AL: "REDUCED POSTPRANDIAL GLUCOSE, GLUCAGON AND TRIGLYCERIDE EXCURSIONS FOLLOWING 4 WEEKS OF PRAMLINTIDE TREATMENT IN PATIENTS WITH TYPE 1 DIABETES TREATED INTENSIVELY WITH INSULIN PUMPS" DIABETES, NEW YORK, NY, US, June 2002 (2002-06), page A117 XP009009880 ISSN: 0012-1797

## Description

### FIELD OF THE INVENTION

The field of the invention is modulation of circulating lipid levels, especially triglyceride levels.

### BACKGROUND

Amylin is a 37-amino acid polypeptide hormone normally co-secreted with insulin by pancreatic beta cells in response to nutrient intake (see, e.g., Koda et al., Lancet 339:1179-1180, 1992). Preclinical studies indicate that amylin acts as a neuroendocrine hormone that complements the actions of insulin in post-prandial glucose control via several effects that collectively reduce the influx of glucose into the circulation to a rate that better matches the rate of insulin-mediated glucose efflux (Weyer et al., Curr Pharm Des 7:1353-73, 2001; Young, Curr Opin Endocrinol Diab 4:282-290, 1997). These effects include a slowing of the rate at which nutrients are delivered from the stomach to the small intestine for absorption (Young et al., Diabetologia 38:642-648, 1995), and a suppression of nutrient-stimulated glucagon secretion (Gedulin et al., Metabolism 46:67-70, 1997). Pramlintide (^{25, 28, 29} Pro-h-amylin) is a synthetic, soluble, non-aggregating analog of human amylin under development as an adjunct to insulin therapy in both type-1 and type-2 diabetes (Weyer et al., Curr Pharm Des 7:1353-73, 2001; Buse et al Clin Diabetes 20: 137-144,2002; Edelman and Weyer, Diabetes Technology and Therapeutics 4: 175-189, 2002). Short-term clinical studies in patients with type-1 diabetes have shown that mealtime amylin replacement with subcutaneous (s.c.) injections of pramlintide, in addition to mealtime insulin, slows the rate of gastric emptying (Kong et al., Diabetologia 41:577-583,1998), suppresses mealtime glucagon secretion (Nyholm et al., Metabolism 48:935-941, 1999; Fineman et al., Metabolism,51:636-641, 2002) and, consequently, improves post-prandial glucose excursions (Nyholm et al., Metabolism 48:935-941, 1999; Thompson et al., Diabetes 46:632-636, 1997).

Abnormalities in circulating lipids have been shown to be associated with increased risk of atherosclerosis and cardiovascular disease. It is known that diabetics and other groups, such as obese individuals, have an increased incidence of lipid abnormalities and an increased risk of cardiovascular morbidity and mortality. These risks may, in part, be related to atherogenic lipid abnormalities, such as an abnormal lipoprotein profile or increased plasma triglyceride concentrations (see, e.g., Georgopoulos Clin. Cardiol., 22:(Suppl. II), II-28-II-33 (1999); Vergès Diabetes & Metabolism 25(Suppl. 3): 32-40 (1999), both herein incorporated by reference). Current therapies exist that primarily target fasting hyperlipidemia, but none to date exist targeting the treatment of post-prandial hyperlipidemia.

### BRIEF DESCRIPTION OF THE FIGURES

Figure 1 shows the amylin agonist pramlintide effectively reducing post-prandial glucose and triglyceride excursions after a standardized mixed meal challenge.
Figure 2 shows the average incremental post-prandial triglyceride concentration in Type 1 diabetes patients using Lispro insulin who received placebo (plac) or pramlintide (pram) at the indicated time points with respect to a standardized meal.
Figure 3 shows the average incremental post-prandial triglyceride concentration in Type 1 patients using regular insulin who received placebo (plac) and pramlintide (pram) at various time points with respect to a standardized meal.
Figure 4 shows the average incremental post-prandial triglyceride concentration in Type 2 diabetes patients with type II diabetes using Lispro insulin receiving placebo (plac) and pramlintide (pram) at various time points with respect to a standardized meal.

### SUMMARY

The present invention provides the use of a composition comprising an amylin or an amylin agonist for the manufacture of a medicament for treating dyslipidemia in a patient, wherein the medicament reduces post-prandial triglyceride excursions and wherein the amylin agonist is an amylin analog or an amylin derivative.

The present invention also provides the use according to claim 1 wherein the medicament reduces circulating lipid levels.

### DETAILED DESCRIPTION

Although amylin and its analogues have been well characterized with respect to regulatory effects on blood glucose levels and various other uses, it is described herein that administration of amylin analogues can be used to beneficially regulate plasma triglyceride concentrations. The reduction of circulating triglycerides can be of benefit to a variety of patients in clinical or veterinary settings. In particular, it is well known that elevated post-prandial triglyceride levels are an independent risk factor for cardiovascular disease. The uses described herein to reduce such levels are thus of considerable clinical importance in improving cardiovascular risk factors.

For example, in healthy human patients, post-prandial triglyceride levels generally do not exceed about 250 mg/dl. However, in certain diseases or disorders, fasting and/or post-prandial levels can greatly exceed this value. This is at least in part attributable to the fact that the capacity to clear triglyceride-rich lipoproteins (and circulating triglycerides) from the circulation is limited in humans, and is especially impaired in people with obesity, insulin resistance, and/ or diabetes, particularly type II diabetes. Hypertriglyceridemia is a clinically important abnormality, since it is associated with an increased risk of cardiovascular disease and related pathologies. For example, patients with type II diabetes mellitus frequently have elevated fasting triglycerides, often on the order of 250-500 mg/dl. Following a meal, circulating triglyceride levels in these patients can rise above 1000 mg/dl, well above the normal range. Such elevated triglycerides are an independent risk factor for atherosclerotic cardiovascular disease, for example, and some studies indicate that the triglycerides of type II patients are more prone to adhere to vascular walls. The methods of the present invention can therefore be used to treat patients with either elevated fasting triglyceride levels, elevated post-prandial triglyceride levels, or both.

It has been discovered that, in addition to glucose-modulating effects, amylin and amylin agonists are effective acutely and/or chronically to maintain or reduce fasting lipid levels, and reduce post-prandial lipid (particularly triglyceride) excursions. This effect is beneficial in reducing cardiac and atherosclerotic risk in patients at higher risk, e.g., those who are genetically predisposed, obese, diabetic, etc. As used herein, "amylin agonists" include amylin, amylin analogs (e.g., having one or more additions or deletions, and/or replacements of amino acids with naturally or non-naturally occurring amino acids such as D-amino acids or peptidomimetics), and amylin derivatives (including but not limited to chemical modifications or additions, acylation, PEGylation, etc.). In a preferred embodiment an amylin agonist is pramlintide. Also described are means of improving the lipid profile in a patient comprising administering an effective amount of an amylin or amylin agonist. As used herein, "lipid profile" means the balance, proportion, or actual concentration of circulating lipids, including triglyceride levels, HDL, LDL, cholesterol, etc. The invention is useful for lowering triglyceride levels in a patient comprising administering an effective amount of an amylin or amylin agonist. Overall lipid or triglyceride levels can be reduced with this method, e.g., fasting levels, post-prandial peak levels, and overall post-prandial lipid/triglyceride level excursions (e.g., as measured by area under the curve (AUC) of post-prandial triglyceride increase compared to the increase in a non-amylin agonist treated state). Individual clinically relevant measures, such as fasting lipid levels (including triglyceride, cholesterol, HDL, and LDL, etc.) and post-prandial lipid (e.g., triglyceride) levels are also reduced by the uses of the invention. Patients having dyslipidemia or altered lipid levels as compared to normal can be treated by administration of amylin or amylin agonists. Diabetic and obese patients, as well as those who are genetically predisposed to dyslipidemia or cardiovascular disease, are particularly suited to treatment by the methods of the invention.

The uses of the invention, involving administration of an amylin or amylin agonist to reduce triglyceride levels, are of benefit to patients having elevated triglyceride levels for any reason. Amylins, amylin agonists, and amylin analogues, as well as doses for use in the invention, are described, for example, in issued US Patent Nos. 6,143,718, 6,087,334, 5,998,367, 5,686,411, 5,367,052, 5,503,989, 5,124,314 and patents referenced therein. Preferred amylin agonists are compounds of the following formula: 
¹A₁ —X—Asn—Thr—⁵Ala—Thr—Y—Ala—Thr—¹⁰Gln—Arg— Leu—B₁ —Asn—¹⁵Phe—
Leu—C₁—D₁—E₁—²⁰F₁—G₁— Asn—H₁ —Gly—²⁵Pro—I₁ —Leu—Pro—J₁ — ³⁰Thr—K₁ —
Val — Gly —Ser—³⁵Asn—Thr—Tyr—Z    (SEQ ID NO 1)
 wherein
A₁ is Lys, Ala, Ser or hydrogen;
B₁ is Ala, Ser or Thr;
C₁ is Val, Leu or Ile;
D₁ is His or Arg;
E₁ is Ser or Thr;
F₁ is Ser, Thr, Gln or Asn;
G₁ is Asn, Gln or His;
H₁ is Phe, Leu or Tyr;
I₁ is Ile, Val, Ala or Leu;
J₁ is Ser, Pro or Thr;
K₁ is Asn, Asp or Gln;
X and Y are independently selected amino acid residues having side chains which are chemically bonded to each other to form an intramolecular linkage, wherein said intramolecular linkage is a disulfide bond, a lactam or a thioether linkage; and Z is amino, alkylamino, dialkylamino, cycloalkylamino, arylamino, aralkylamino, alkyloxy, aryloxy or aralkyloxy; and provided that when A₁ is Lys, B₁ is Ala, C₁ is Val, D₁ is Arg, E₁ is Ser, F₁ is Ser, G₁ is Asn, H₁ is Leu, I₁ is Val, J₁ is Pro, and K₁ is Asn; then one or more of A₁ to K₁ is a D-amino acid and Z is selected from the group consisting of alkylamino, dialkylamino, cycloalkylamino, arylamino, aralkylamino, alkyloxy, aryloxy or aralkyloxy.

A particularly preferred amylin agonist is pramlintide (^{25, 28, 29} Pro-h-amylin). Other preferred amylin agonists include des-¹Lys-h-amylin, ²⁵Pro²⁶Val^{28,29}Pro-h-amylin, ¹⁸Arg^{25,28}Pro-h-amylin, des-¹Lys¹⁸Arg^{25,28}Pro-h-amylin, ¹⁸Arg^{25,28,29}Pro-h-amylin, des-¹Lys ¹⁸Arg^{25,28,29}Pro-h-amylin, ^{25,28,29}Pro-h-amylin, des-¹Lys^{25,28,29}Pro-h-amylin, ²⁵Pro²⁶Val^{28,29}Pro-h-amylin, ²⁸Pro-h-amylin, ^{2,7}Cyclo-[²Asp,⁷Lys]-h-amylin, ²⁻³⁷h-amylin, ¹Ala-h-amylin, ¹Ser-h-amylin, ²⁹Pro-h-amylin, ^{25,28}Pro-h-amylin, des-¹Lys^{25,28}Pro-h-amylin, ²³Leu²⁵Pro²⁶Val^{28,29}Pro-h-amylin, ²³Leu²⁵Pro²⁶Val²⁸Pro-h-amylin, des-¹Lys²³Leu²⁵Pro²⁶Val²⁸Pro-h-amylin, ¹⁸Arg²³Leu²⁵Pro²⁶Val²⁸Pro-h-amylin, ¹⁸Arg²³Leu^{25,28,29}Pro-h-amylin, ¹⁸Arg²³Leu^{25,28}Pro-h-amylin, ¹⁷Ile²³Leu^{25,28,29}Pro-h-amylin, ¹⁷Ile^{25,28,29}Pro-h-amylin, des-¹Lys ¹⁷Ile²³Leu^{25,28,29}Pro-h-amylin, ¹⁷Ile¹⁸Arg²³Leu-h-amylin, ¹⁷Ile¹⁸Arg²³Leu²⁶Val²⁹pro-h-amylin, ¹⁷Ile¹⁸Arg ²³Leu²⁵Pro²⁶Val^{28,29}Pro-h-amylin, ¹³Thr²¹His²³Leu²⁶Ala²⁸Leu²⁹Pro³¹Asp-h-amylin, ¹³Thr²¹His ²³Leu²⁶Ala²⁹Pro³¹Asp-h-amylin, des-¹Lys ¹³Thr²¹His²³Leu²⁶Ala²⁸Pro³¹Asp-h-amylin, ¹³Thr¹⁸Arg ²¹His²³Leu²⁶Ala²⁹Pro³¹Asp-h-amylin, ¹³Thr¹⁸Arg ²¹His²³Leu^{28,29}Pro³¹Asp-h-amylin, and ¹³Thr¹⁸Arg²¹His²³Leu²⁵Pro²⁶Ala^{28,29}Pro³¹Asp-h-amylin.

Suitable doses can additionally be determined by a clinical practitioner using known parameters, without undue experimentation. Variables to be taken into account include the patient's weight, physical status, the potency of the agonist being used, etc. In addition to those disclosed herein, dosing regimens such as those provided in Diabetes Technol Ther 2002, 4(1):51-61; Diabetes Care 2002, 25(4):724-730; Diabetes 1997, 46(4):632-6; Diabet Med 1997, 14(7):547-55; Diabetes Care 1998, 21(6):987-93; Diabetes 2000, 49(1):A109; Diabetes 2000, 49(suppl 1):A106; Diabetes Care 2003,26(1):1-8; Diabetes Technol Ther 2002, 4(2):175-189; or Clin Diabetes 2002, 20:137-144 can be used with the methods of the present invention.

The present invention can also be used to treat patients suffering from atherosclerosis with or without elevated cholesterol levels. Although applicants do not wish to be bound by theory, it is believed that this benefit is caused by the effect of lowered triglyceride levels on the ability of lipoprotein lipase to clear triglycerides from the peripheral circulation. Lipoprotein lipase, in addition to its function of catalyzing the breakdown of circulating triglycerides, catalyzes the conversion of very low density lipoprotein (VLDL) to intermediate density lipoproteins (IDLs), which are subsequently converted to high density and low density lipoproteins (HDLs and LDLs). As circulating triglyceride levels are lowered, lipoprotein lipase is made available to further catalyze IDL to the point that the IDL is removed by the liver for conversion to LDL and/or HDL, which can then be appropriately sequestered in the tissues. This can result in a net improvement in overall cholesterol/lipid levels in a patient. Methods of the present invention therefore include a method to maintain or lower cholesterol levels in a patient, and particularly in a patient having high cholesterol or atherosclerosis/atherogenesis. Patients other than those suffering from diabetes can also be treated with the present invention. Means maintaining or lowering the level of IDLs in a patient are also described.

As used herein, "treating elevated triglyceride levels in a patient" means either preventing an increase in those levels or causing a reduction in those levels relative to the level prior to treatment.

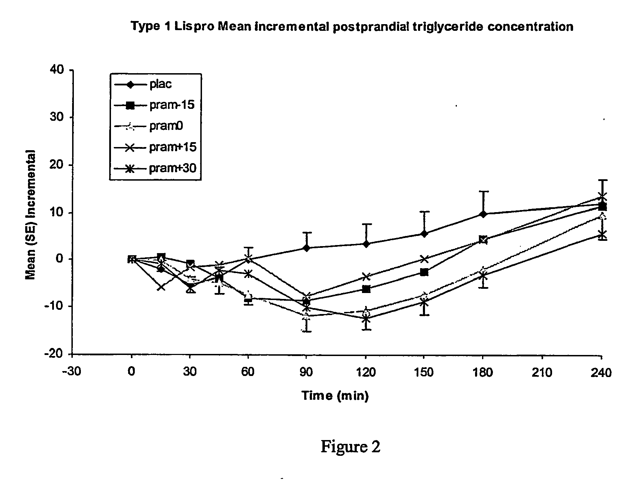

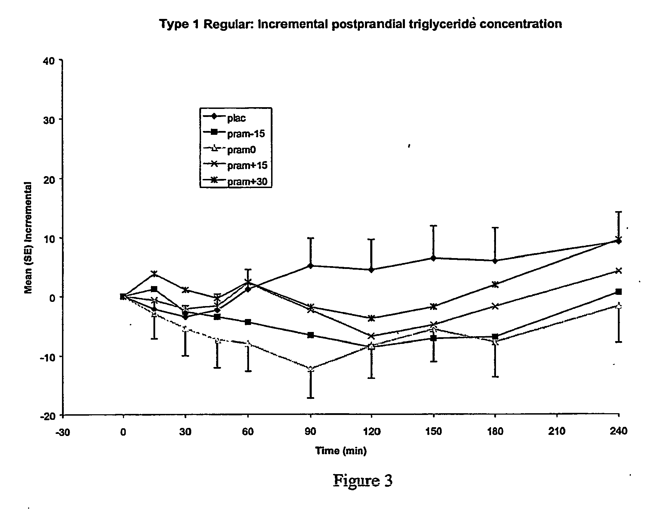

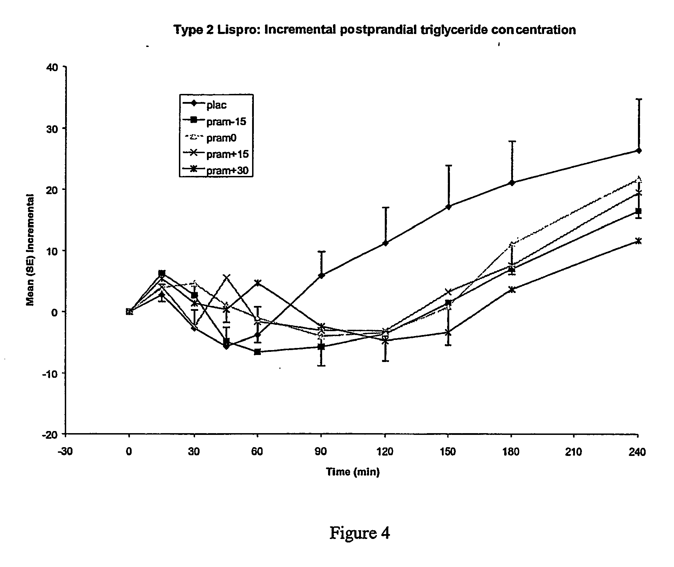

As used herein, "reducing post-prandial triglyceride excursions" means lowering the both the peak concentration and the total area under the triglyceride concentration curve that is seen in patients after eating a meal. This typically will mean lowering the total area under the curve for a graph such as those provided in figures 2-4 within the hour following a meal.

As used herein "reducing circulating lipid levels" in a patient means lowering the measurable amount of blood lipids relative to the level before treatment.

As used herein, "treating dyslipidemia" means improving or restoring to a level, ratio, profile, or balance closer to that medically defined as normal and/or healthy any or all lipid or lipoprotein parameters clinically measurable. This includes, but is not limited to, levels of triglycerides, LDL, HDL, IDL, VLDL, total cholesterol, apolipoproteins, etc..

As used herein, "improving circulating lipid profile in a patient" means causing a change in concentration of one or more lipids found in blood in order to change the overall blood lipid content of the patient to a preferred state. It can also include shifting the distribution of lipids over different lipoprotein fractions, without changing the overall content/concentration of circulating lipids.

As used herein, "treating hypertriglyceridemia" in a patient means causing a lowering in concentration of triglycerides found in the blood of the patient at one or more relevant times, for example while fasting or post-prandial.

As used herein "reducing post-prandial circulating triglycerides" in a patient means lowering the measurable amount of triglycerides in the circulation after a meal (e.g., in the period about 4-6 hours after the meal) relative to the level of such lipids seen post-prandially in the patient before or without treatment for a similar meal.

Preferred doses for the uses of the invention include administering to the patient an amount of amylin or amylin agonist between about 0.125 µg/kg/dose and about 5.0 µg/kg/ dose, or between about 0.5 µg/kg/ dose and about 4.0 µg/kg/ dose, or between about 1.0 µg/kg/ dose and about 3.0 µg/kg/ dose. In a preferred embodiment a patient suffering from type I diabetes or having a body mass index (BMI) below about 28-30 m²/kg is given a dose of about 5.0 to about 90.0 µg/dose, more preferably about 10.0 to about 70.0 µg/dose, and more preferably about 30.0 to about 60.0 µg/dose. In a preferred embodiment a patient suffering from type II diabetes or having a BMI at or above about 30m²/kg is given a dose of about 20.0 to about 360.0 µg/dose, or about 90.0 to about 240.0 µg/dose, or preferably about 50.0 to about 120.0 µg/dose. Preferred times for administration are around any bout of eating, preferably major or high fat meals. Preferred administration times include about -120, -60, -45, -30, -15, 0, 15, 30, 45, 60, and 120 minutes relative to the meal time, preferably about -60 to 60 min of the meal, more preferably - 15 min before to about 15 minutes after the meal. Depot or sustained release formulations will obviously not be administered around meals per se, but levels of the amylin or amylin agonist will be present at these preferred times. For non-depot formulations, generally about 1-5 doses will be administered per day. Administration need not be associated with all meals, but is preferably associated with large or high fat meals.

Any means of administration known in the art may be employed to deliver the amylin or amylin agonist of the invention. For example, injection, oral, nasal, peripheral, pulmonary, transdermal, transmucosal, or continuous infusion (e.g., via a pump or an implantable reservoir or matrix) delivery may be used (see e.g., Remington: The Science and Practice of Pharmacy, 19th ed. (1995) Mack Publishing Company, Easton, Pa.; herein incorporated by reference). Preferred administration is via subcutaneous administration or sustained release formulation.

Short or long term administration of the amylin or amylin agonist is contemplated, as effects are seen with a single dose in reducing plasma lipid levels. However, preferably multiple doses are given over a period of time (e.g., longer than one week) for increased and sustained efficacy in lowering plasma/circulating lipids.

The following Examples illustrate certain aspects of the invention, and are not intended to be limiting in any way.

### Example 1

### This study assesses the effect of the amylin agonist pramlintide on post-prandial plasma glucose and triglyceride excursions in patients with type-1 diabetes intensively treated with continuous subcutaneous insulin infusion (CSII).

All 23 patients in this study are ≥16 years of age, have type-I diabetes for ≥1 year and have been intensively treated (CSII, basal/bolus regimen) for at least 6 months. Patients are using either lispro (n=21) or regular (n=2) insulin, have not changed their daily insulin dose by more than ±10% for 2 months prior to the study, and have been free from severe hypo- and hyperglycemic symptoms for at least 4 weeks prior to the study. Exclusion criteria include a clinically significant history or presence of heart disease, uncontrolled hypertension (blood pressure ≥160/90 mm Hg), gastrointestinal, hepatic, renal, or central nervous system disorders, acute illness, a history of drug or alcohol abuse, or treatment with drugs known to affect gastrointestinal motility or glucose metabolism.

The study consists of 3 time periods (baseline, 4 weeks on-therapy, 2 weeks off-therapy). At the end of each time period (weeks 0, 4, 6), patients are evaluated at the clinical research center (CRC). Each evaluation includes a standardized meal test.

The first evaluation (baseline) is conducted when patients are solely treated with their usual CSII regimen. After the baseline evaluation, patients are randomized to self-administer either placebo (n=6) or pramlintide (n=17) TID with major meals, in addition to their existing CSII therapy, for 4 weeks. The placebo group is included to provide data that could be used to determine adequate power analyses when planning future studies and not for the purpose of placebo comparisons. Study medication is self-administered within 15 minutes prior to major meals and is injected s.c. into the anterior abdominal wall opposite the insertion site of the CSII catheter. To minimize the risk of hypoglycemia upon initiation of pramlintide treatment, all patients are instructed to reduce their preprandial insulin doses by 10-20% during the first 3 days of therapy and to subsequently titrate their insulin dose as clinically indicated. Patients record their daily insulin regimen throughout the study. The insulin dose regimens, along with the blood glucose self-monitoring results, are reviewed by the investigator at each visit and, consistent with good medical practice, adjusted as deemed appropriate.

Three days prior to the end of the 4-week on-therapy period, subjects return to the Clinical Research Center (CRC) to repeat the standardized meal test while still receiving study medication. Subjects then return to their pre-existing therapy (CSII only) and, after 2 weeks, return to the CRC for the third standardized meal test (off-therapy period).

On each of the 3 CRC visits, patients undergo a standardized mixed meal test the morning after an overnight fast. The meal consists of 1 bagel, 1 cheese slice, orange juice, margarine, and 2% milk (20% of total daily caloric requirements; 55%, 15%, and 30% of calories derived from carbohydrate, protein, and fat, respectively) and is completely ingested within 5 minutes. Patients receive a s.c. injection of study medication (pramlintide or placebo) 15 min prior to the meal and administer their preprandial insulin dose immediately prior to the meal. Blood samples are collected at -30, -15, 30, 60, 90, 120, and 180 minutes relative to the standardized meal for determination of plasma glucose and triglyceride concentrations. Blood samples are processed in accordance with standard clinical practice using a reference lab and commercially available products (e.g., quantitative enzymatic *in vitro* assay for Triglycerides GPO-PAP, Roche Diagnostics).

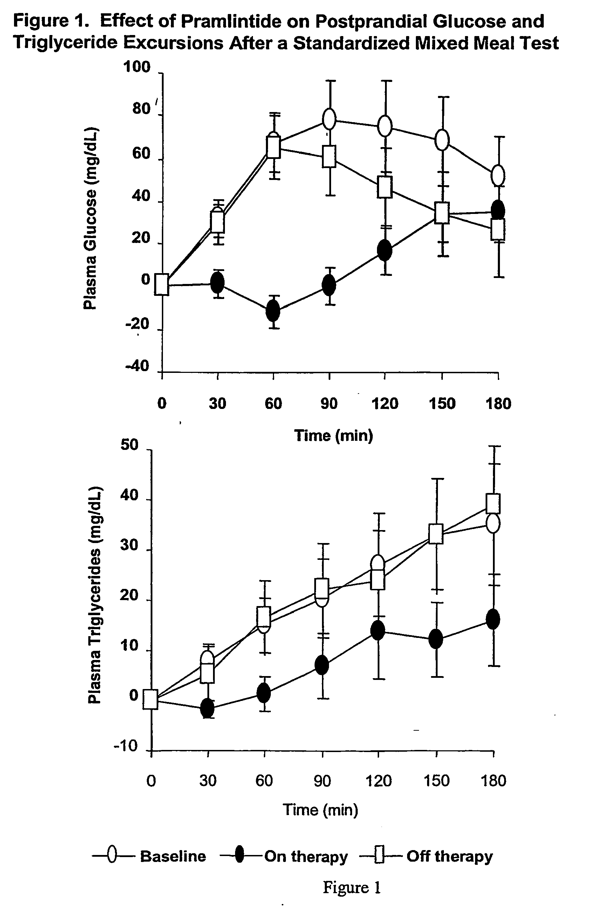

The main outcome variable for the standardized meal test are the changes from baseline in the incremental area under the curve (AUC) for glucose and triglyceride concentrations over time during the baseline, on-therapy, and off-therapy in the pramlintide treatment group. During the baseline period, patients exhibit post-prandial triglyceride excursions (relative to fasting concentrations), with a mean incremental area under the curve (AUC) of 65.7 ± 87.6 mg•dL⁻¹•min (Fig 1). At the end of the 4-week pramlintide-treatment period, post-prandial triglyceride excursions improve markedly, with a mean incremental AUC of 16.7 ± 56.8 mg•dL⁻¹ •min (p <0.05) (Fig 1). At week 6, 2 weeks after subjects return to their pre-treatment therapy (CSII only), post-prandial triglyceride excursions return toward baseline values, with a mean incremental AUC of 56.2 ± 87.3 mg•dL⁻¹•min (Fig 1).

The data show that the addition of pramlintide to insulin reduces not only the post-prandial glucose response, but also the post-prandial triglyceride response to a mixed meal in type-1 diabetic patients. Alternative administration protocols could alter the fasting triglyceride levels, for example, using infusion or long-acting formulation, or multiple dosing regimens. Although the exact mechanism underlying the observed reduction of the post-prandial triglyceride response with pramlintide has not yet been elucidated and applicants do not wish to be bound by theory, it is possible that the effect of pramlintide to slow gastric emptying plays an important role. By slowing the delivery of ingested nutrients (carbohydrates, lipids, and proteins) from the stomach to the small intestine, pramlintide likely redresses not only the inflow of glucose into the circulation, but also the inflow of chylomicrons and other meal-derived lipoproteins (1,7). This helps to better match the rate of lipoprotein clearance, thereby limiting the post-prandial triglyceride excursions. It is noteworthy that the observed post-prandial triglyceride effect in this study occurs in patients with relatively low fasting triglyceride levels and in response to a meal with relatively low fat content. The post-prandial lipid-lowering effects of pramlintide or other amylin agonists will be more pronounced in patients having a diet containing a larger lipid load, or in hyperlipidemic patients (e.g., type II diabetics). This post-prandial lipid-lowering effect offers an important additional clinical benefit of pramlintide, given that many type-1 and type-2 diabetic patients experience post-prandial hyper- and dyslipidemia (6,8), which are well-established cardiovascular risk factors.

### Example 2

In this example, a randomized, single-blind, placebo-controlled, five-way crossover study is designed to examine the effect of the timing of pramlintide injection relative to meal ingestion on postprandial plasma glucose profiles and triglycerides in subjects with type 1 diabetes and subjects with type 2 diabetes using insulin. Three study groups are defined by diabetes type and mealtime insulin used in their treatment regimen. On 5 study days, subjects receive a single dose of one of five treatments (A, B, C, D, or E) in random order:

| **Treatment** | **Study** | **Timing Relative to a** |
|---|---|---|
| | **Medication** | **Standardized Breakfast** |
| A | Placebo | - 15 minutes |
| B | Pramlintide | - 15 minutes |
| C | Pramlintide | 0 minutes |
| D | Pramlintide | + 15 minutes |
| E | Pramlintide | + 30 minutes |

Each treatment (pramlintide or placebo) is administered subcutaneously (SC) within specified times relative to a standardized breakfast after an overnight fast. Subjects are randomly assigned to one of four treatment sequences according to a randomization schedule generated for each study group.

Subjects with type 1 diabetes received pramlintide 60 µg or placebo (equivalent volume). Subjects with type 2 diabetes using insulin receive pramlintide 120 µg or placebo (equivalent volume). Subjects maintain their usual insulin regimen during the study; if necessary, insulin dose adjustments are made in consultation with study personnel to facilitate achievement of fasting fingerstick glucose values in the range of ≥80 mg/dL and ≤250 mg/dL while avoiding hypoglycemia. The timing of the short-acting insulin dose relative to the standardized breakfast is based on recommendations in the respective package inserts for the insulin (0 min for insulin lispro and -30 min for regular insulin).

The study population consists of three study groups, defined by diabetes type and type of mealtime insulin used in their treatment regimen. Study Group 1: Subjects with type 1 diabetes using insulin lispro. Study Group 2: Subjects with type 1 diabetes using regular insulin. Study Group 3: Subjects with type 2 diabetes using insulin lispro. Fifty-nine subjects (21 type 1 subjects using insulin lispro; 19 type 1 subjects using regular insulin; and 19 type 2 subjects using insulin lispro) are enrolled in this study.

The groups have the following characteristics. Type 1 Subjects Using Insulin Lispro: Of the 21 randomized subjects, 8 (38.1%) are female and 13 (61.9%) are male. The majority of subjects are Caucasian (61.9%), and the mean age of the subject population is 40.8 years. Across treatment sequences, the mean duration of diabetes ranges from 17.1 years to 24.2 years; the mean HbA_{1c} is 8.1% to 8.7%; and the mean BMI ranges from 24.9 kg/m² to 27.2 kg/m². In general, baseline characteristics appear to be similar across treatment sequences.

Type 1 Subjects Using Regular Insulin: Of the 19 randomized subjects, 5 (26.3%) are female and 14 (73.7%) are male. The majority of subjects are Caucasian (57.9%), and the mean age of the subject population is 37.3 years. Across treatment sequences, the mean duration of diabetes ranges from 15.3 years to 28.8 years; the mean HbA_{1c} ranges from 9.0% to 9.6%; and the mean BMI ranges from 25.0 kg/m² to 28.0 kg/m². With the exception of duration of diabetes, baseline characteristics appear similar across treatment sequences.

Type 2 Subjects Using Insulin Lispro: Of the 19 randomized subjects, 10 (52.6%) are female and 9 (47.4%) are male. The majority of subjects are Hispanic (52.6%), and the mean age of the subject population is 49.6 years. Across treatment sequences, the mean duration of diabetes ranges from 8.2 years to 26.6 years; the mean HbA_{1c} ranges from 8.7% to 10.2%; and the mean BMI ranges from 31.0 kg/m² to 37.4 kg/m². With the exception of duration of diabetes, HbA_{1c}, and BMI, baseline characteristics appear to be similar across treatment sequences.

Mealtime amylin replacement via subcutaneous injections of pramlintide just prior to and up to 30 minutes after the meal markedly improves postprandial glucose excursions, and postprandial triglyceride concentrations in type 1 patients treated with either lispro or regular insulin, and in type 2 patients treated with lispro insulin, as shown in Figures 2, 3, and 4.

The figures show that mealtime amylin replacement via subcutaneous injections of pramlintide just prior to and up to 30 minutes after a meal markedly improve post-prandial glucose excursions, and post-prandial triglyceride concentrations in type 1 patients treated with either lispro or regular insulin, and in type 2 patients treated with lispro insulin.
1. Kong MF, Stubbs TA, King P, Macdonald IA, Lambourne JE, Blackshaw PE, Perkins AC, Tattersall RB: The effect of single doses of pramlintide on gastric emptying of two meals in men with IDDM. Diabetologia 41:577-583, 1998
2. Nyholm B, Orskov L, Hove KY, Gravholt CH, Moller N, Alberti KG, Moyses C, Kolterman O, Schmitz O: The amylin analog pramlintide improves glycemic control and reduces postprandial glucagon concentrations in patients with type 1 diabetes mellitus. Metabolism 48:935-941, 1999
3. Fineman M, Koda JE, Shen LZ, Strobel SA, Maggs D, Weyer C, Kolterman O: The human amylin analogue, pramlintide, corrects postprandial hyperglucagonemia in patients with type I diabetes. Metabolism 51(5):636-641, 2002
4. Thompson RG, Peterson J, Gottlieb A, Mullane J: Effects of pramlintide, an analog of human amylin, on plasma glucose profiles in patients with IDDM: Results of a multicenter trial. Diabetes 46:632-636, 1997
5. Whitehouse F, Kruger DF, Fineman M, Shen L, Ruggles JA, Maggs DG, Weyer C, Kolterman OG: A randomized study and open-label extension evaluating the long-term efficacy of pramlintide as an adjunct to insulin therapy in type 1 diabetes. Diabetes Care 25(4):724-730, 2002
6. Georgopoulos A. Postprandial triglyceride metabolism in diabetes mellitus. Clin Cardiol 22(6 Suppl):II28-33, 1999
7. Weyer C, Maggs DG, Young AA, Kolterman OG: Amylin replacement with pramlintide as an adjunct to insulin therapy in type 1 and type 2 diabetes mellitus: a physiological approach toward improved metabolic control. Curr Pharm Des 7:1353-73, 2001
8. Verges BL. Dyslipidaemia in diabetes mellitus. Review of the main lipoprotein abnormalities and their consequences on the development of atherogenesis. Diabetes & Metab 25(Suppl 3):32-40, 1999

The preceding description and Examples are intended to be illustrative. Those skilled in the art to which the invention pertains will appreciate that alterations and changes in the described protocols may be practiced without departing from the scope of this invention.

## Claims

1. Use of a composition comprising an amylin or an amylin agonist for the manufacture of a medicament for treating dyslipidemia in a patient, wherein the medicament reduces post-prandial triglyceride excursions and wherein the amylin agonist is an amylin analog or an amylin derivative.

2. Use according to claim 1 wherein the medicament reduces circulating lipid levels.

3. Use according to claim 1 wherein the medicament improves a circulating lipid profile.

4. Use of a composition comprising an amylin or an amylin agonist for the manufacture of a medicament for treating hypertriglyceridemia in a patient, wherein the medicament reduces post-prandial triglyceride excursions and wherein the amylin agonist is an amylin analog or an amylin derivative.

5. Use according to claims 1-4, wherein one or more lipid levels are elevated during periods of fasting and/or post-prandial periods.

6. Use according to claim 5, wherein the lipid level is the level of triglycerides, HDL, LDLor VLDL.

7. Use according to claims 5 and 6, wherein the lipid is triglycerides.

8. Use according to claims 1-7, wherein the medicament is formulated to administer amylin or an amylin agonist at a dose of between about 0.125 µg/kg/dose and about 5.0 µg/kg/dose.

9. Use according to claims 1-7, wherein the medicament is formulated to administer amylin or an amylin agonist at a dose of between 0.5 µg/kg/dose and about 4.0 µg/kg/dose.

10. Use according to claim 1, wherein the amylin analog is pramlintide.

11. Use according to claims 1-10, wherein the patient has diabetes mellitus.

12. Use according to claims 1-11, wherein the patient is at a higher than average risk for cardiovascular disease.

13. Use according to claims 1-12, wherein the patient is obese.

## Patentansprüche

1. Verwendung einer Zusammensetzung umfassend einen Amylin oder einen Amylin-Agonisten zur Herstellung eines Medikaments zur Behandlung von Dyslipidämie bei einem Patienten, wobei das Medikament Abweichungen des post-prandialen Triglycerids reduziert, und wobei der Amylin-Agonist ein Amylin-Analog oder ein Amylin-Derivat ist.

2. Verwendung nach Anspruch 1, **dadurch gekennzeichnet, dass** das Medikament in Zirkulation befindliche Lipidspiegel reduziert.

3. Verwendung nach Anspruch 1, **dadurch gekennzeichnet, dass** das Medikament ein Profil des zirkulierenden Lipids verbessert.

4. Verwendung einer Zusammensetzung umfassend ein Amylin oder einen Amylin-Agonisten zur Herstellung eines Medikaments zur Behandlung von Hypertriglyceridämie bei einem Patienten, wobei das Medikament Abweichungen des post-prandialen Triglycerids reduziert, und wobei der Amylin-Agonist ein Amylin-Analog oder ein Amylin-Derivat ist.

5. Verwendung nach den Ansprüchen 1 bis 4, **dadurch gekennzeichnet, dass** ein oder mehrere Lipidspiegel während Perioden des Fastens und/oder post-prandialen Perioden erhöht sind.

6. Verwendung nach Anspruch 5, **dadurch gekennzeichnet, dass** der Lipidspiegel der Spiegel an Triglyceriden, HDL, LDL oder VLDL ist.

7. Verwendung nach den Ansprüchen 5 oder 6, **dadurch gekennzeichnet, dass** das Lipid Triglyceride sind.

8. Verwendung nach den Ansprüchen 1 bis 7, **dadurch gekennzeichnet, dass** das Medikament zur Verabreichung von einem Amylin oder einem Amylin-Agonisten in einer Dosis von zwischen etwa 0,125 µg/kg/Dosis und etwa 5,0 µg/kg/Dosis formuliert ist.

9. Verwendung nach den Ansprüchen 1 bis 7, **dadurch gekennzeichnet, dass** das Medikament zur Verabreichung von Amylin oder einem Amylin-Agonisten in einer Dosis von zwischen 0,5 µg/kg/Dosis und etwa 4,0 µg/kg/Dosis formuliert ist.

10. Verwendung nach Anspruch 1, **dadurch gekennzeichnet, dass** das Amylin-Analog Pramlintid ist.

11. Verwendung nach den Ansprüchen 1 bis 10, **dadurch gekennzeichnet, dass** der Patient Diabetes mellitus hat.

12. Verwendung nach den Ansprüchen 1 bis 11, **dadurch gekennzeichnet, dass** der Patient ein höheres als durchschnittliches Risiko für kardiovaskuläre Erkrankungen aufweist.

13. Verwendung nach den Ansprüchen 1 bis 12, **dadurch gekennzeichnet, dass** der Patient fettleibig ist.

## Revendications

1. Utilisation d'une composition comprenant une amyline ou un agoniste d'amyline pour la fabrication d'un médicament destiné à traiter une dyslipidémie chez un patient, dans laquelle le médicament réduit les excursions post-prandiales des triglycérides et dans laquelle l'agoniste d'amyline est un analogue d'amyline ou un dérivé d'amyline.

2. Utilisation selon la revendication 1, dans laquelle le médicament réduit les taux de lipides en circulation.

3. Utilisation selon la revendication 1, dans laquelle le médicament améliore le profil des lipides en circulation.

4. Utilisation d'une composition comprenant une amyline ou un agoniste d'amyline pour la fabrication d'un médicament destiné à traiter l'hypertriglycéridémie chez un patient, dans laquelle le médicament réduit les excursions post-prandiales des triglycérides et, dans laquelle l'agoniste d'amyline est un analogue d'amyline ou un dérivé d'amyline.

5. Utilisation selon les revendications 1-4, dans laquelle un ou plusieurs taux de lipides sont élevés pendant les périodes de jeûne et/ou les périodes post-prandiales.

6. Utilisation selon la revendication 5, dans laquelle le taux de lipides est le taux de triglycérides, de HDL, de LDL ou de VLDL.

7. Utilisation selon les revendications 5 et 6, dans laquelle le lipide est des triglycérides.

8. Utilisation selon les revendications 1-7, dans laquelle le médicament est formulé pour que l'amyline ou un agoniste d'amyline soit administré à une dose comprise entre environ 0,125 µg/kg/dose et environ 5,0 µg/kg/dose.

9. Utilisation selon les revendications 1-7, dans laquelle le médicament est formulé pour que l'amyline ou un agoniste d'amyline soit administré à une dose comprise entre 0,5 µg/kg/dose et environ 4,0 µg/kg/dose.

10. Utilisation selon la revendication 1, dans laquelle l'analogue d'amyline est le pramlintide.

11. Utilisation selon les revendications 1-10, dans laquelle le patient est attein de diabète sucré.

12. Utilisation selon les revendications 1-11, dans laquelle le patient présente un risque supérieur à la moyenne de maladie cardiovasculaire.

13. Utilisation selon les revendications 1-12, dans laquelle le patient est obèse.
